# EUROPEAN PATENT APPLICATION

(11) **EP 4 819 155 A1**
(43) Date of publication of application: **07.10.2026**
(21) Application number: 26170645.1
(22) Date of filing: 02.04.2026
(51) Int. Cl.: G16H 40/67, A61B 5/00, G06F 11/20, G16H 40/63, G06F 3/16

(54) **SYSTEMS AND METHODS FOR MIRRORING AN INTERFACE OF A MEDICAL DEVICE**

(30) Priority: 03.04.2025 US 202563782829 P; 01.04.2026 US 202619636715
(71) Applicant: STRYKER CORPORATION, Portage, MI 49002-9711 (US)
(72) Inventor: STAMNES, Mark Edward, Portage, 49002-9711 (US); LOSQUADRO, Matthew Robert, Portage, 49002-9711 (US); GREENEWALD, Robert, Portage, 49002-9711 (US); DURAI, Sathiyan, Portage, 49002-9711 (US); LIU, Michelle, Portage, 49002-9711 (US); BOEHM, Ethan, Portage, 49002-9711 (US); CHESTER, Steven Mark, Portage, 49002-9711 (US)
(74) Representative: Kilburn & Strode LLP

(57) **Abstract**

An example system includes an external device and a monitor-defibrillator. The monitor-defibrillator may include a detection circuit configured to detect an electrical signal indicative of an electrocardiogram (ECG) of a subject, a transceiver, a display and a processor configured to determine that the display is unable to visually present the ECG. In response to determining that the display is unable to visually present the ECG, the transceiver can transmit, to the external device, an instruction to display the ECG such that the ECG is mirrored via an external display of the external device.

## Description

### CROSS-REFERENCE TO RELATED APPLICATIONS

This application claims priority to U.S. Provisional Application No. 63/782,829, titled "SYSTEMS AND METHODS FOR MIRRORING AN INTERFACE OF A MEDICAL DEVICE," which was filed on April 3, 2025, and to US Nonprovisional Application No. 19/636,715, having the same title, which was filed on April 1, 2026.

### BACKGROUND

Medical devices can be used to monitor patients and/or to administer treatments to patients. Medical devices used in emergency situations may have a display that presents physiological data (e.g., heart rate, electrocardiogram, temperature, etc.). However, it can be difficult for a group of people to view the display from a distance simultaneously. Additionally, if the display of the medical device is broken, cracked, or otherwise obscured, the medical device can lose its utility.

### BRIEF DESCRIPTION OF THE DRAWINGS

FIG. 1 illustrates an example environment including an example medical device with a malfunctioning display and an example external device that is configured to mirror the display of the example medical device.
FIG. 2 illustrates an example environment including example external devices configured to display physiological data presented via a display of the example medical device.
FIG. 3 illustrates an example environment including an example external device configured to present physiological data received from multiple monitoring devices.
FIG. 4 illustrates example environment including example components of a medical device and example components of an external device configured to perform various functions described herein.
FIG. 5 illustrates an example process for determining a display of a medical device has a malfunction and causing an external device to mirror the display of the medical device.
FIG. 6 illustrates an example process for receiving, from an external device, a request to mirror a display of a medical device and transmitting, to the external device, an instruction to mirror the display via an interface associated with the external device.

### DETAILED DESCRIPTION

Medical personnel (e.g., paramedics or other rescuers, doctors, nurses etc.) rely on medical devices having displays to present data in order to properly treat and monitor a condition of a subject (e.g., a patient). However, the display on a medical device may be difficult to see from a distance or by a group of personnel (e.g., due to the medical device having a small display). That is, the display may not readily be visible to caregivers. Additionally, in some instances, a display of a medical device may malfunction, making the medical device practically unusable. For example, the display of a medical device may be cracked or shattered (e.g., due to the medical device being dropped, crushed, punctured, etc.), present distortions (e.g., due to damaged pixels), become unresponsive or otherwise freeze (e.g., due to damaged components such as a display driver), or malfunction for various reasons discussed herein.

The techniques, devices and systems described herein enable a medical device to transmit display data to one or more external devices. This enables multiple individuals to monitor the same data presented on the display of the medical device. Additionally, if the display of the medical device malfunctions (e.g., a cracked display, damage to a circuit configured to power the display, etc.), the medical device may transmit display data to an external device, thus enabling the medical device to still be utilized. In some instances, the display data may be streamed to an external device in real-time or near-real time, enabling the medical device to still be used even if the display is malfunctioning or is damaged. Mirroring the display of the medical device allows the display data to be duplicated and presented via an external display.

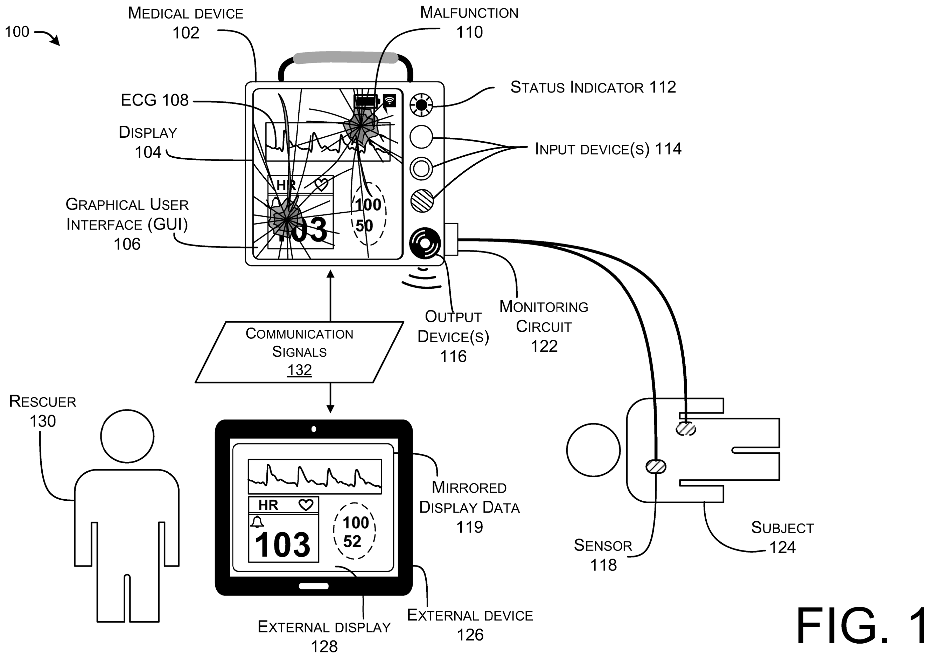

FIG. 1 illustrates an example environment 100 including an example medical device 102 with a display 104 having a malfunction 110. An external device 126 is configured to mirror the display 104 of the example medical device 102 via an external display 128. As illustrated in FIG. 1, the environment 100 includes a rescue scene. In some examples, the rescue scene may be in a clinical environment (e.g., a hospital) or a non-clinical environment (e.g., the scene of an accident). In some cases, the subject 124 may be experiencing cardiac arrest, respiratory arrest, or some other dangerous medical condition. The subject 124 may be monitored or treated by a rescuer 130 (e.g., an emergency medical technician (EMT) professional, doctor, nurse etc.) utilizing one or more monitoring devices or medical devices. In some examples, additional medical devices may be utilized in conjunction with the medical device 102 in order to assist with the monitoring and treatment of the subject.

According to some implementations, the medical device 102 may be a defibrillator. For example, the medical device 102 may be an external defibrillator, such as an automated external defibrillator (AED) or a monitor-defibrillator. In some examples, the medical device 102 may be a portable medical device. A rescuer 130 may utilize the medical device 102 to evaluate, treat, or monitor a condition of the subject 124. In some examples, the rescuer 130 may utilize additional medical device(s) to evaluate, treat, or monitor the subject 124. For example, the rescuer 130 may utilize one or more of an additional monitor-defibrillator, a medical imaging device, an ultrasound monitor, a standalone electrocardiogram (ECG) monitor, chest compression device, ventilator, pulse oximeter, capnograph, continuous glucose monitor (CGM), portable X-ray machine, a ventilation device, or any other medical device configured to assess, treat, or monitor a subject. Although illustrated in FIG. 1 as separate individuals, in some cases, the subject 124 is the rescuer 130.

The medical device 102 (e.g., a monitor-defibrillator) may include a display 104, status indicator 112, input device(s) 114, output device(s) 116, sensor 118, a monitoring circuit 122, or any combination thereof.

The monitoring circuit 122 is communicatively coupled to a sensor(s) 118. Sensor 118 may include, for example, at least one of an electrode, a detection circuit, defibrillator pads, a force sensor, a blood pressure cuff, an ultrasound-based blood pressure sensor, an invasive (e.g., intra-arterial) blood pressure sensor (e.g., including a cannula inserted into the subject 124), a gas sensor (e.g., a carbon dioxide, oxygen sensor, etc.), a flowmeter, a pulse oximetry sensor, a regional oximetry sensor, thermometer, microphone, etc. In some examples, the monitoring circuit 122 includes one or more ports configured to receive analog signals indicative of the physiological parameters. The one or more ports can be electrically coupled to one or more sensors (e.g., at least one parameter sensor). In at least one example, the monitoring circuit 122 may be configured to determine that the display 104 has a malfunction, determine that the transceiver is communicatively coupled to the external device, and cause the transceiver to transmit an instruction to visually present display data (e.g., a physiological parameter configured to be presented via display 104) via an interface of the external device.

The sensor 118 is configured to detect at least one physiological parameter of the subject 124. As used herein, the term "physiological parameter," and its equivalents, may refer to an indication of a subject's health including, for instance, an ECG, an impedance, a force administered to the subject 124, a blood pressure, an airway parameter (e.g., a partial pressure of carbon dioxide, a partial pressure of oxygen, a capnographs parameters, an end-tidal carbon dioxide (EtCO₂), a flow rate, etc.), a blood oxygenation (e.g., a pulse oximetry value, a regional oximetry value, etc.), an electroencephalogram (EEG), a temperature, a heart sound, a blood flow rate, a physiological geometry (e.g., a shape of a blood vessel, an inner ear shape, etc.), a heart rate, a pulse rate, an airway parameter, a plethysmograph, or the like. For example, the sensor 118 includes at least one of electrodes, a detection circuit, defibrillator pads, a force sensor, a blood pressure cuff, an ultrasound-based blood pressure sensor, an invasive (e.g., intra-arterial) blood pressure sensor (e.g., including a cannula inserted into the subject 124), a gas sensor (e.g., a carbon dioxide, oxygen sensor, etc.), a flowmeter, a pulse oximetry sensor, a regional oximetry sensor, a thermometer, a microphone, an ultrasound transducer, and the like.

In various cases, the medical device 102 outputs the physiological parameter(s) to the rescuer 130. For instance, the medical device 102 includes a display(s) 104, a speaker(s), or haptic feedback device(s) that conveys an alert associated with a physiological parameter(s) to the rescuer 130. The alert, for instance, may indicate that at least one physiological parameter is outside of a predetermined range (e.g., bounded by thresholds). In some cases, the medical device 102 generates the alert in response to detecting signs of a condition, such as a cardiac arrhythmia, based on an analysis of the physiological parameter(s). In the example of FIG. 1, the medical device 102 is outputting, via a display 104 of the medical device 102, an ECG 108 parameter.

The medical device 102 may include input device(s) 114 and output device(s) 116. The input device(s) 114 and the output device(s) 116 function as an interface between a user and the medical device 102. The input device(s) 114 is configured to receive an input from a user and can include a keypad, a cursor control, a touch-sensitive display, a voice input device (e.g., a microphone), or any combination thereof. The output device(s) 116 includes at least one of a display, a speaker, a haptic feedback device, a printer, a transceiver, or any combination thereof. In some implementations, the input device(s) 114 includes one or more touch sensors, and the output device(s) 116 includes a display screen, and the touch sensor(s) are integrated with the display screen. The input device(s) 114 may include a physical input device such as a button, a knob, a switch, a dial, a slider, and the like.

The display 104 is configured to graphically present data via a graphical user interface (GUI) 106. The data can include physiological parameters, alerts, a status of the device (e.g., battery status 206), messages, instructions, recommendation, notification (e.g., a notification indicating a detected artifact, types of artifacts, lead(s) affected, and electrodes causing a problem, etc.). FIG. 1 illustrates the GUI 106 presenting an ECG 108 of the subject 124. In some examples, the display 104 may be configured to present physiological parameters in a manner that is easily digested and understood while also conveying a reliability of such data, without filling or cluttering a display with background or additional graphics. For instance, the GUI 106 may omit icons or other graphics portraying some data that would be utilized by trained medical providers using the medical device 102 in a manual mode to evaluate the data on the display 104.

Various types of data (or image data) may be presented via the GUI 106 associated with the display 104. For example, display data may include physiological data (e.g., cardiovascular data such as heart rate, blood pressure, electrocardiogram, arrhythmia data, etc.), respiratory data (e.g., respiratory rate, oxygen saturation, tidal volume, etc.), neurological data (e.g., electroencephalogram data, intracranial pressure, etc.), metabolic data (e.g., blood glucose level), temperature, and the like. Display data may also include medical device operational data indicating a status of the medical device, such as battery level, charging status, device settings and configuration (e.g., mode of operation, parameter settings, etc.), connectivity and communication status (e.g., network status), and the like. Display data may also include alerts and messages configured to be presented via the display 104, such as system errors and fault codes, sensor malfunction alerts, maintenance alerts, patient health alerts (e.g., cardiac arrest or irregular heartbeat alert, etc.), treatment and therapy alerts (e.g., defibrillator readiness warning due to low battery), or any other type of data configured to be presented via the display 104.

In some instances, the display 104 may be unable to visually present data. For example, the display 104 may have a malfunction 110. The malfunction 110 may include, for example, a backlight malfunction, discoloration of the display, freezing of the display, flickering of the display, damage of a circuit configured to power the display, a cracked display, or an electromagnetic interference. In some examples, the malfunction 110 may be caused in part by malfunction of the touch sensors of a display 104. The malfunction 110 may be caused by a hardware fault and/or a software fault of the medical device 102. In some examples, a malfunction detection component (e.g., malfunction detection component 422) associated with the medical device 102 may be configured to determine that the display 104 is unable to visually present data (e.g., an ECG) or otherwise has a malfunction. The malfunction detection component 422 is described in further detail below.

In some examples, in response to determining that the display 104 has the malfunction 110, a transceiver of the medical device 102 may transmit data to an external device 126 for display via an external display 128. The external device 126 may be, for example, a laptop, tablet, smartphone, desktop computer, smartwatch, a television (e.g., a smart TV), a monitor, a projector, and the like.

Upon connecting to the external device 126, a status indicator 112 disposed on the medical device 102 may indicate a connection status. That is, the status indicator 112 may include a light source (e.g., light emitter) configured to output a light signal indicating the medical device 102 is communicatively coupled to and is transmitting communication signals 132 to external device 126. In some examples, an output device(s) 120 associated with the medical device 102 may emit an audible tone indicating that the medical device 102 is communicatively coupled to the external device 126.

In various implementations of the present disclosure, the medical device 102 is configured to communicate with external devices. For example, the medical device 102 is configured to transmit and receive data from an external device 126 configured to transmit and receive data. In some examples, the display data may be transmitted over one or more wireless communication links (e.g., a WI-FI^{®} link, a WIGIG^{®} link, a BLUETOOTH^{®} link, a radio link, a near field communication (NFC) link, or the like), one or more wired communication links (e.g., an Ethernet link, an optical fiber link, or the like), or a combination thereof. In some cases, the data is transmitted over one or more communication networks, such as a local area network (LAN), a wide area network (WAN) (e.g., the Internet), a mobile core network (e.g., a 3_{rd} Generation Partnership Project (3GPP) network), a radio access network (RAN), or any combination thereof.

The medical device 102 may be configured to establish a communication link with the external device 126. In some examples, establishing the communication link may include causing a transceiver of the medical device to output a pairing request to the external device. In at least one example, the pairing request may be output in response to determining that the display 104 has a malfunction 110. The medical device 102 may determine that the transceiver has received a pairing response from the external device. In response to receiving the pairing response from the external device 126, the medical device 102 can establish a communication link between the transceiver and the external device 126. The communication link may be a bi-directional communication link that enables both devices to send and receive data.

According to various examples, the external device 126 includes an external display 128 configured to output the display data in any manner equivalent to the display 104 of the medical device 102. For example, the medical device 102 transmits image data indicative of the GUI 106, (e.g., user interface elements, the ECG 108, heart rate, etc.) to an external device 126, thereby causing the external device 126 to output any portion of display data on its respective display. In some examples, the medical device 102 may transmit (e.g., via a transceiver) display data to a remote location, such as to an ambulance that is traveling to an emergency scene, or a remote clinical environment (e.g., hospital) that may be expecting the subject.

Once the medical device 102 is communicatively coupled to the external device 126, the medical device 102 may transmit communication signals 132 (e.g., data stream including digital or analog information) to external device 126. The communication signals 132 may include display data of the medical device. The mirrored display data 119 can be streamed image data from the medical device 102. That is, the streamed image data can be indicative of the physiological parameters generated at the medical device 102. The display data may include user interface elements or any other data displayed on the display 104. This can enable multiple rescuers (or caregivers) to view the display of the medical device 102. In some instances, remote computing devices not present at the scene (e.g., computing device in an ambulance, at a hospital, etc.) can communicatively couple to the medical device 102 and/or external device 126 and view the display data. In some instances, if the display 104 of the medical device 102 has a malfunction, the medical device 102 may still be usable by a rescuer to administer treatment and monitor physiological parameters of a subject as other components of the medical device may be undamaged.

In some examples, the medical device 102 may be configured to transmit communication signals 132 to an external device upon detecting the external device 126 is within a threshold distance of the medical device 102. In this example, the external device 126 may have previously been authenticated or authorized to receive communication signals 132 from the medical device 102. In some instances, the external device 126 can stream display data received from the medical device in response to determining a latency condition is met (e.g., latency between about 100-1000 milliseconds (ms)). In at least one example, the external device 126 may detect the latency drop below a latency threshold (e.g., latency longer than 1 second, 2 seconds, etc.). In some instances, in response to determining the latency condition is not met, the external device 126 may decrease a resolution of the received display data in order to improve or increase a transmission time.

In some examples, the external display may enable the rescuer 130 to interact with the medical device 102 via the external display 128. For example, the rescuer 130 may interact with user interface elements presented via the external display and the external device 126 can transmit communication signals 132 to the medical device 102.

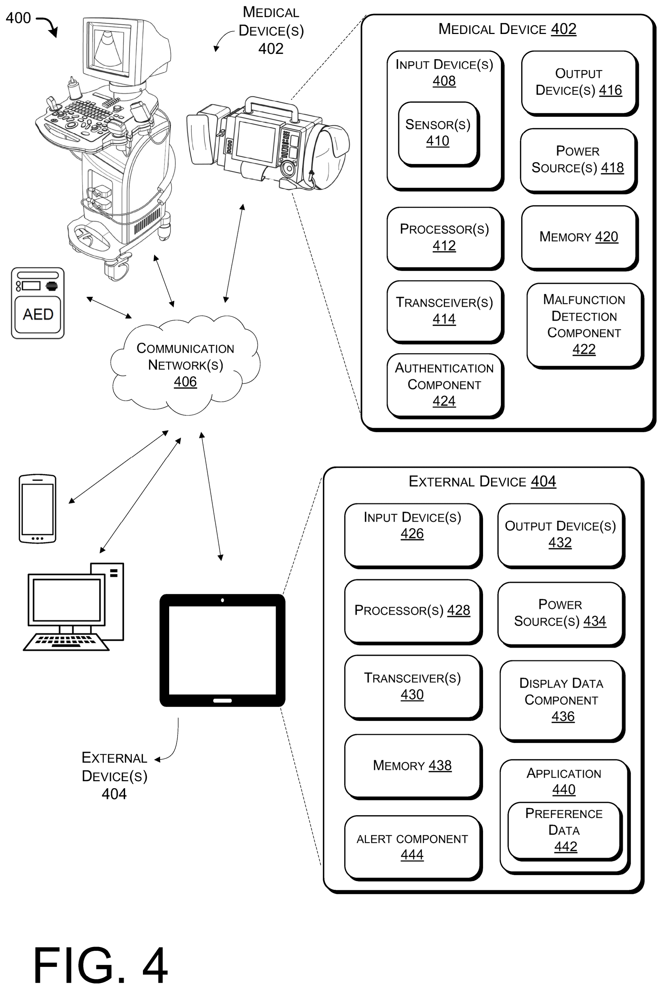

Referring now to FIG. 1, one exemplary operation of the present embodiment will be described. A subject 124 may be experiencing cardiac arrest. A rescuer 130 may arrive at the scene with a medical device 102 (e.g., a monitor defibrillator). However, display 104 of the medical device 102 may be damaged or have a malfunction. For example, the display 104 in FIG. 1 is illustrated as being cracked at multiple areas of the display 104. This may be due to the medical device 102 being accidently dropped, compressed, or an object may have been dropped onto the display 104, etc. The malfunction 110 may prevent data from being viewed via the display 104. For example, the heart rate and the ECG data presented on display 104 may be partially obscured, which can affect the monitoring or treatment of the subject 124. In some examples, the display 104 may be frozen due to malfunction 110. A malfunction detection component (e.g., malfunction detection component 422 discussed below in relation to FIG. 4) may be configured to detect and determine that the display 104 has a malfunction 110. In response to detecting the malfunction 110, the medical device 102 may initiate pairing with an external device 126 having an external display 128. In at least one example, the external device 126 may have previously been authorized to receive data from the medical device 102 (e.g., due to previously being paired to the medical device 102). A communication link may be established between the medical device 102 and the external device 126. The medical device 102 may transmit (e.g., via communication signals 132) display data (e.g., image data) to external device 126. The external device 126 may be configured to present the display data via an external display 128 as illustrated in FIG. 1. That is, the display data can be duplicated or mirrored via the external display 128 (e.g., mirrored display data 119), enabling the rescuer 130 to monitor and treat the subject 124 while still using the medical device 102.

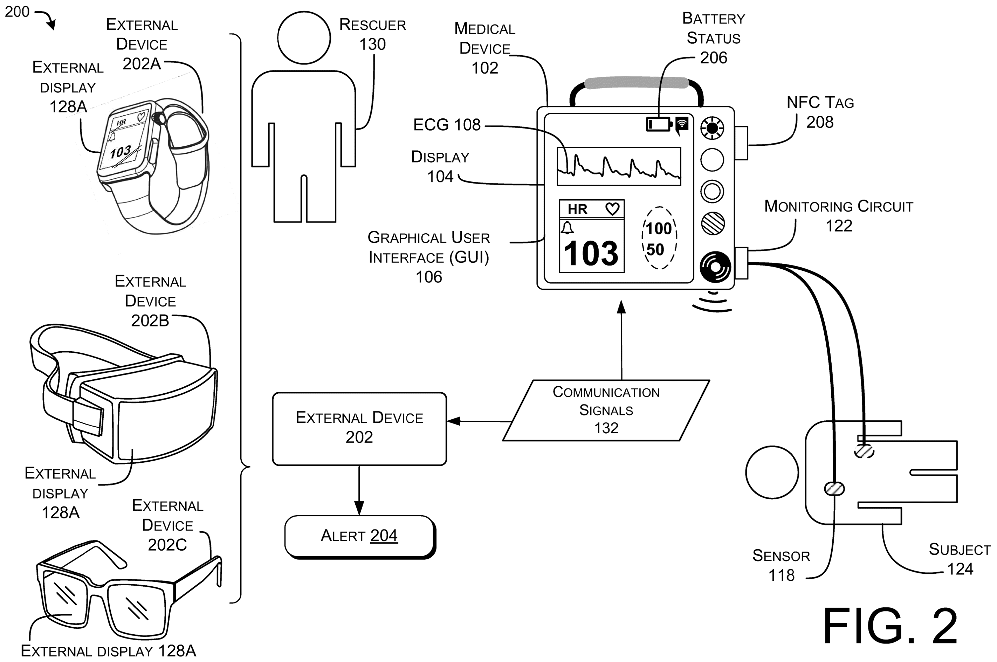

FIG. 2 illustrates an example environment 200 including example wearable external devices configured to visually present display data generated at the medical device 102. In some examples, the external device 202 may be a wearable device such as a smartwatch (first wearable external device 202A), virtual reality headset (e.g., second wearable external device 202B), head mounted display, augmented reality glasses (third wearable external device 202C), and the like.

The medical device 102 may include a proximity sensor. The proximity sensor can harness short-range wireless technology to establish a communication with the external device 202. The proximity sensor may be a NFC tag (e.g., NFC tag 208) that uses magnetic field induction (e.g., wireless connectivity standard (Ecma-340, ISO/IEC 18092)) to enable communication between the NFC tag 208 and the external device 202 when touched together or brought within a few centimeters (e.g., four centimeters) of each other. The proximity sensors are in communication with a controller associated with the medical device through wired or wireless means commonly known in the art.

As discussed above, a medical device 102 may present a variety of data via a display 104. For example, the medical device 102 may be configured to display physiological data (e.g., heart rate, blood pressure, respiratory rate, body temperature, oxygen saturation, ECG/EKG reading, pulse rate, blood flow, EEG waveforms, etc.), a status of the medical device 102 (e.g., battery status 206, connection status, error messages, alerts, etc.), and the like. However, not all data presented via the display 104 of the medical device 102 may be equally relevant to a rescuer 130 or another caregiver present at the scene of an accident. For example, the battery status 206 of the medical device 102 may not be as equally relevant as a heart rate of the subject 124. Additionally, in some instances, the display of a wearable device (e.g., smartwatch) may be smaller relative to the display of a medical device (e.g., a monitor-defibrillator). As such, a caregiver may desire to limit the data (or type of data) that is presented via the display of the external device.

In some examples, the communication signals 132 may include display data representing the contents of the display 104. In an alternative example, the communication signals 132 may include a subset of data generated at the medical device 102 (e.g., physiological data and not battery status 206). In some examples, the subset of data may be determined based in part on determining a type of the external device 202 configured to receive the communication signals 132. A type of external device 202 may include a smartphone (e.g., Apple (iOS), Android, etc.), smartwatch (e.g., Apple smartwatch, Android smartwatch, etc.), tablet, laptop, portable media player, E-reader, augmented and virtual reality devices, and the like. In some examples, a type of external device 202 may include a wearable external device type (e.g., smartwatch), external computing devices having screens that are at or below a screen size threshold (e.g., smartphone having a screen at or below about twelve centimeters), external computing devices having screens that are at or above a screen size threshold (e.g., a tablet or laptop that has a screen at or above about twenty-five centimeters), and the like.

In various embodiments, the external device 202 may communicatively couple to the medical device 102 using the proximity sensor. That is, by positioning the external device 202 within a suitable proximity of the NFC tag 214, a rescuer 130 may be automatically presented with display data of the medical device 102 without requiring further navigation on the external device 202. In some examples, the external device 202 may communicatively couple to the medical device 102 using a QR code.

In some examples, the medical device 102 may receive a pairing request from the external device 202 (e.g., via Bluetooth paring, Wi-Fi direct, etc.). The pairing request may include data indicating a type of external device. In response to the pairing request, the medical device 102 may determine a subset of data to transmit to the external device for display. In at least one example, the subset of data may be determined at the external device level. For example, the communication signals 132 may include the totality of the display data presented via display 104 and the external device 126 may be configured to determine the subset of display data to be presented via external display 128. In some instances, the subset of data output via external display 128 is determined by a size of the display associated with the external device 202.

The external device 202 may be associated with an application (e.g., application 440 discussed below) configured to receiving and present data. The application can enable a user to specify what data (or types of data) to present via a display of the external device 202. The application may assist with a caregiver's duties by displaying a portion of the display data of the medical device 102. As a non-limiting example, a user of an external device 202 may subscribe to heart rate data and not ECG data. FIG. 2 illustrates the first external device 202A displaying a heart rate that is also displayed via display 104, but not the ECG 108 data.

In some examples, the application may be associated with a user profile (or account). In some instances, the user profile may be associated with the external device. The user profile may include preference data indicating what display data is to be presented to the user via the external device 202. User preferences may include for example, an alert preference (e.g., alert sound, alert type, visual alert, vibration or haptic feedback alert, recurring alarm, etc.), a physiological parameter preference (e.g., subscribe to receiving heart rate, blood pressure, ECG data, etc.), and the like. In some examples, a user may subscribe to receive physiological data associated with one or more individuals (e.g., patients). For example, a user (e.g., caregiver) may subscribe to receiving physiological data associated with patients on a particular floor of a hospital, patients that are in a critical condition (e.g., patients in an intensive care unit), and the like. The subscription settings can be edited via the application as needed. In some examples, the external device may be associated with default user settings. Default user settings can include pre-configured settings of the application. The pre-configured settings may be associated with a user profile when an application is installed or first launched.

In some embodiments, an application may be integrated into the present embodiment. For example, the external device 202 may be associated with an application that enables a user to display data received from a medical device (or multiple medical devices), In some examples, the application enables a user to view display data from multiple medical devices treating and monitoring multiple subjects. The application may allow a user to change how much and/or what portion of the display of the medical device to present via the external display. In at least one example, when the external device is within a suitable proximity to the NFC tag 214, an application may be opened that enables the user to remotely view the display data.

In some examples, the application may be designed or customized to the operational functions of the medical device 102 and the external device 202. For example, an external device 202 may be associated with a device type (e.g., handheld device, wearable device, head-up display, etc.). Depending on the type of device communicatively coupled to the medical device 102, a subset of display data may be presented via the external display. For example, a handheld computing device having a larger sized display, such as a laptop or a tablet, may be configured to mirror the display 104 because of the larger sized display. A smartwatch, on the other hand, may be configured to selectively present a portion of the data presented via display 104, such as physiological data and not alerts, messages, or data indicating a status of the medical device, etc.

In some examples, the external device may include an alert component (e.g., alert component 444 discussed below in relation to FIG. 4) that is configured to provide feedback to a rescuer 130. An alert can be associated with one or more feedback types, such as haptic feedback (e.g., vibrations, pulses, pressure changes or constriction of a band, etc.), audible feedback (e.g., notification sound or alarm), acoustic feedback, and/or visual feedback (e.g., a message to be presented via a display). The alert component may be configured to generate an alert 204 in response to detecting a trigger event. In some examples, a trigger event may include detecting a physiological parameter that is outside a predetermined physiological parameter range (e.g., a heart rate is below or above a threshold heart rate, blood oxygen levels are below a predetermined threshold, irregular heart rhythm is detected, etc.). In some instances, a trigger event may include detecting a condition or malfunction of the medical device (e.g., display malfunction, low battery, detected blockage in the tubing of an IV pump, electrode pad disconnection, etc.). In some examples, the medical device 102 may be associated with a separate alert component configured to generate a separate alert. This allows multiple rescuers or caregivers to be alerted as to a condition of the subject 124.

In some examples, the external device 202 may generate an alert 204 that is of the same type or a different type than an alert generated by the medical device 102. For example, in response to detecting a trigger event, the medical device 102 may generate an audible alert (e.g., notification sound, alarm, etc.) while the external device 202 may be configured to generate an alert 204 having a haptic feedback type (e.g., a smartwatch may generate a vibration to alert the rescuer 130 to a condition of the subject or the medical device). This can decrease a number of alerts that are generated at a scene, which may be distracting and may even interfere with the effectiveness of patient care during an emergency.

In some examples, the external device 202 generates an alert 204 in response to determining a physiological parameter of the subject 124 has met or exceeded a first threshold and the medical device 102 may generate a separate alert in response to determining that a physiological parameter has met or exceeded a second threshold different than the first threshold. For example, the external device 202 may generate an alert 204 when a heart rate of a subject 124 exceeds 200 beats per minute (bpm) while the medical device 102 may be configured to generate an alert when a heart rate of the subject exceeds 150 bmp (e.g., a lower threshold).

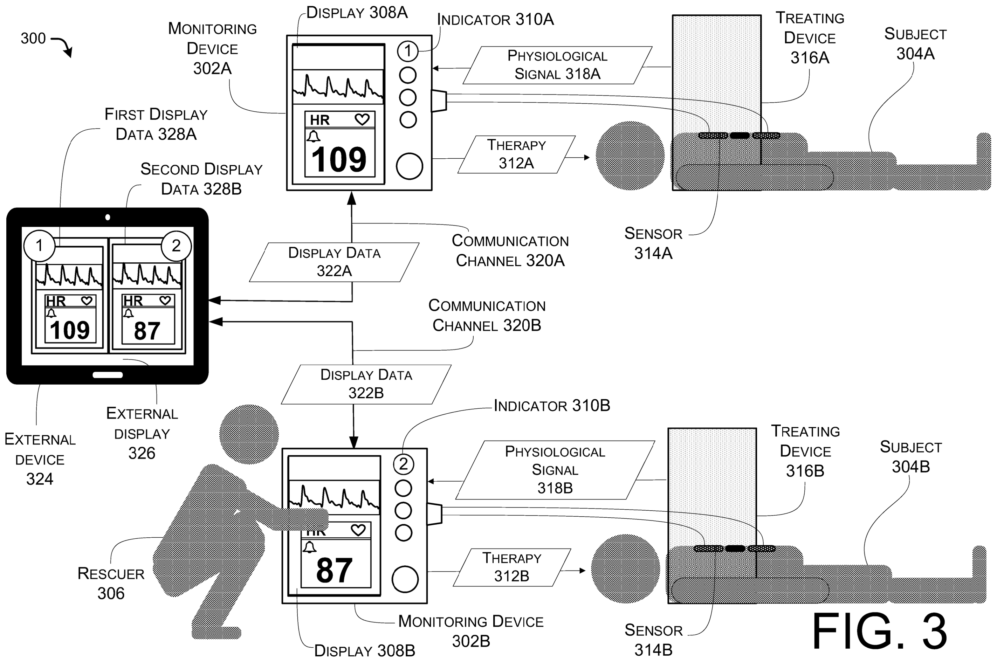

FIG. 3 illustrates an environment 300 including an example external device configured to present physiological data received from multiple monitoring devices at a rescue scene. The example environment 300 may include multiple casualties (or patients), each of which may be treated with one or more monitoring devices (e.g., medical devices). As such, the external device 324 may be configured to communicatively couple to multiple monitoring devices that are treating multiple subjects. The rescue scene, in some implementations, is within a clinical environment, such as a hospital or other managed care facility.

In some cases, the rescue scene is outside of a clinical environment. In various implementations, a rescuer 306 is treating a first subject 304A and a second subject 304B at the rescue scene. For example, the rescuer 306 is an EMT professional monitoring and treating medical conditions of the subjects.

The rescuer 306 is monitoring the condition of the first subject 304A and using a first monitoring device 302A and a second monitoring device 302B. The first monitoring device 302A may have a first display 308A and the second monitoring device 302B may have a second display 308B. In some examples, the monitoring devices may be a monitor-defibrillator, a medical imaging device, an ultrasound monitor, a standalone electrocardiogram (ECG) monitor, or another type of patient monitor. The first monitoring device 302A and the second monitoring device 302B and each communicatively coupled to sensors (e.g., first sensor 314A and second sensor 314B). The sensors are configured to detect at least one physiological parameter of the subject, such as those described above in relation to FIG. 1. In various cases, the monitoring devices output the physiological parameter to the rescuer 306. (e.g., via an external display 326, a speaker, or haptic feedback device that conveys the physiological parameter(s) to the rescuer 306).

A treating device (e.g., first treating device 316A and second treating device 316B) (also referred to as "treatment devices") is configured to administer a treatment (e.g., first therapy 312A and second therapy 312B) to a first subject 304A and a second subject 304B, respectively. A treating device can include a monitor-defibrillator, an AED, mechanical chest compression device, a smart bag-valve mask, a ventilator, a heart-lung machine, or the like. Examples of treatments (e.g., therapies) include defibrillation, pacing, cardioversion, administration of chest compressions, administration of oxygen to the airway of a subject, movement of air in the airway of the subject, administration of fluids to the subject. extracorporeal membrane oxygenation (ECMO), administration of a medication to the subject, or the like. In some implementations, the monitoring device is also configured to administer a treatment to the subject. Further, in some cases, the treating device is configured to detect one or more physiological parameters of the subject. The treating device can then send a physiological signal (e.g., first physiological signal 318A and second physiological signal 318B) to the monitoring device.

In some cases, the monitoring and treatment of the subject is optimized by communication between the monitoring device (e.g., first monitoring device 302A or second monitoring device 302B) and the treating device (e.g., first treating device 316A or second treating device 316B). In particular examples, the monitoring device or the treating device reports a detected physiological parameter to the other device. For instance, the treating device may include a blood pressure sensor and may report a blood pressure of the patient to the monitoring device. In some cases, the monitoring device or the treating device reports a treatment parameter to the other device. For example, the treating device may report, to the monitoring device, a frequency of chest compressions administered by the treating device. The receiving device may perform one or more actions based on the physiological parameter or the treatment parameter. Actions performed by the monitoring device or the treating device can include initiating a measurement of a physiological parameter at a particular time or frequency, outputting a signal to the rescuer 306, outputting a signal to the subject, generating an instruction for performing a treatment at a particular time or frequency, generating an instruction for adjusting a treatment parameter of an ongoing treatment, or any combination thereof. According to some implementations, the monitoring device or the treating device transmits an instruction the other device to perform one or more actions. The receiving device is configured to perform the action(s) based on the instruction from the monitoring device or the treating device. The receiving device can generate an internal control signal to perform the action(s) based on the instruction from the monitoring device or the treating device. For instance, the monitoring device instructs the treating device to cease administering chest compressions to the patient at a predetermined time, and the monitoring device administers a defibrillation shock to the subject at the particular time. By exchanging reports, instructions, or other data, the monitoring device and the treating device can coordinate monitoring and treatment of the subject.

To exchange data, the monitoring device or the treating device are configured to communicate via a communication channel between one another. As used herein, the term "communication channel," and its equivalents, may refer to a medium over which a first endpoint (e.g., a sender) transmits information to one or more second endpoints (e.g., receivers). Examples of communication channels include wired connections, such as Ethernet or fiber optic paths, as well as wireless connections, such as Institute of Electronics and Electrical Engineers (IEEE) (e.g., WI-FI, BLUETOOTH, etc.) or 3rd Generation Partnership Program (3GPP) (e.g., Long Term Evolution (LTE), New Radio (NR), etc.) connections. As used herein, the term "endpoint," and its equivalents, may refer to an entity that is configured to transmit and/or receive data. Examples of endpoints include user equipment (UE) (e.g., mobile phones, tablet computers, etc.), computers, base stations, access points (APs), servers, compute nodes, medical devices, Internet of Things (IoT) devices, and the like.

In some implementations, the communication channel between the monitoring device and the treating device is established when the monitoring device and the treating device are paired. In particular examples, the monitoring device and treating device refrain from sharing substantive data (e.g., physiological metrics, reports about the patient, instructions for treating the patient, etc.) until the monitoring device and the treating device are paired. As used herein, the term "paired," and its equivalents, may refer to a state of multiple devices that have a shared link key that enables each device to cryptographically authenticate data it receives from any other device among the multiple devices.

In some examples, the monitoring device and/or treating device establishes a communication channel (e.g., first communication channel 320A and a second communication channel 320B) with an external device 324. Various mechanisms can be utilized to pair the monitoring devices with the external device 324. For example, automated pairing may involve the exchange of data and pairing requests/responses between the devices. As another example, the monitoring device may receive an input signal from an operator (e.g., the rescuer 306) that selects the external device 324 as a device to pair with the monitoring device, or vice versa. In some cases, the monitoring device detects an alternative signal (e.g., a flashing light pattern) from the external device 324 that is indicated in a pairing request. In some cases, the monitoring device and the external device 324 are paired, at least in part, based on signaling to and from an intermediary device (not illustrated in FIG. 3). In a particular example, two or more devices can be brought into proximity to (e.g., into contact with) each other in order to facilitating pairing the monitoring device. For example, a "tap-to-pair" functionality may allow a user to bring the monitoring device (or a component thereof) into close proximity to (e.g., into contact with) the external device 324 (or a component thereof), or vice versa, and a short-range wireless protocol, such as BLUETOOTH, NFC, or the like, may be used to detect that the devices are within a threshold distance of each other, and, in response, the devices may be paired.

In particular examples, a paired monitoring device (e.g., first monitoring device 302A or second monitoring device 302B) encrypts data (e.g., first display data 322A and second display data 322B) prior to transmitting the data to external device 324 to be displayed via external display 326. The external device 324 may be configured to restore the original data by decrypting the encrypted data. As used herein, the term "encrypt," and its equivalents, refers to a process of translating data from one format (e.g., an unencoded format) into an encoded format. In various cases, the encoded format is referred to as "ciphertext." Unencoded data, which has not been encrypted, may be referred to as being in "plaintext." In various examples, an entity encrypts data using at least one encryption key. An encryption key is a parameter that defines the translation of data from the one format into the encoded format. As used herein, the term "decrypt," and its equivalents, refers to a process of translating data from an encoded format into another format (e.g., an unencoded format), such as a plaintext format. In various examples, an entity encrypts data using at least one decryption key. A decryption key is a parameter that defines the translation of data from the encoded format into the other format. A link key, for example, is an encryption and/or decryption key.

Various cryptographic techniques can be utilized in accordance with the features described in this disclosure. For example, data can be encrypted and decrypted via a symmetric key, wherein the encryption key and the decryption key are equivalent. In some cases, data can be encrypted and decrypted via asymmetric keys, wherein the encryption key and the decryption key are different. Cryptographic hash functions (CHFs) are examples of cryptographic techniques. Examples of cryptographic techniques include the Data Encryption Standard (DES), Advanced Encryption Standard (AES), Elliptic Curve Cryptography (ECC), Rivest-Shamir-Adleman (RSA), Secure Hash Algorithm (SHA)-1, SHA-2, SHA-3, BLAKE, BLAKE2, BLAKE3, WHIRLPOOL, MD2, MD4, MD5, MD6, Temporal Key Integrity Protocol (TKIP), Rivest cipher 4 (RC4), variably modified permutation composition (VMPC), blowfish, Twofish, Threefish, Tiny Encryption Algorithm (TEA), Extended TEA (XTEA), Corrected Block TEA (XXTEA), Diffie-Hellman exchange (DHE), elliptic curve DHE, supersingular isogeny Diffie-Hellman (SIDH) key exchange, and so on. Any suitable encryption or decryption technique can be used in accordance with implementations of this disclosure.

The external device 324 may be configured to mirror display data (e.g., first display data 322A, second display data 322B, etc.) of the monitoring devices. That is, the screen(s) of the monitoring device(s) can be duplicated via the external device 324. This may enable the rescuer 306 to monitor multiple monitoring devices and/or multiple subjects. The display data from multiple devices may be displayed via a single screen (e.g., the same GUI) of the external display 326. For example, the external device 324 presents the first display data 322A and the second display data 322B within two sections of the screen display (e.g., split-screen view) such that multiple displays can be mirrored via a single display. Alternatively, the external device 324 may present data from multiple devices and/or multiple subjects using a carousel view (e.g., content appears in a sequence and can be scrolled through), tabbed view (e.g., content is divided into separate tabs or pages), continuous view (e.g., content can be fluidly scrolled through where different sections load as a user swipes the screen), and the like.

To differentiate between display data presented via a single external display, the monitoring devices may be associated with indicators that identify the monitoring device upon pairing. An identifier may be a code (e.g., number, letter, or a combination thereof), an emitter (e.g., a light emitter that emits a particular color), and the like. For example, the first monitoring device 302A is associated with a first indicator 310A that identifies the first monitoring device 302A with a number "1" while the second monitoring device 302B is associated with a second indicator 310B that identifies the second monitoring device 302B with a number "2." In some examples, the first indicator 310A may additionally or alternatively include a first light emitter that emits a first color (e.g., red) while the second indicator 310B may additionally or alternatively include a second light emitter that emits a second color (e.g., green). These identifiers are then displayed via the external display 326 proximate their respective display data such that the rescuer 306 can quickly and easily identify which display data corresponds to which monitoring device.

In some examples, the first display data 328A and the second display data 328B may indicate one or more of a patient's name, location (e.g., room number), or other specific patient information. In some examples, the first display data 328A or the second display data 328B may be organized and presented via the external display 326 in order of need for medical attention or listed in some other order, such as based on when each monitoring device was communicatively coupled to the external device 324. If a single rescuer 306 is monitoring more than one patient simultaneously, the external device 324 may be configured to present display data based on a ranking of urgency between patients, depending on the patient's physiological parameters or symptoms.

In some examples, the environment 300 may be hospital environment that includes nursing stations. A nursing station may include multiple patient monitors. In some instances, individual nurses may carry or wear an external device that enables the nurse (or other caregiver) to view the display data from multiple monitoring devices.

Caregivers, such as medical doctors, nurses, etc., are then able to simultaneously monitor patients from a single external device that they can wear (e.g., smartwatch, head-up display device, etc.). The external device 324 may be configured to provide alerts (alarms) to let the caregiver know the status of the patient and determine which patient(s) need to be attended to. In certain examples, the external device 324 may display a patient's name, room number (location) and other specific patient information in association with the display data being mirrored from the monitoring device. In certain examples, either through preset thresholds that are set at via an application associated with individual external devices 324, display data may be presented or otherwise organized in order of need for medical attention or listed some other order, such as by a time the external device was paired to or otherwise accessed the display data of a monitoring device. The external device 324 is configured to monitor more than one patient simultaneously and the external device may provide a ranking of urgency between patients, depending on a patient's symptoms.

In certain embodiments, the monitoring device may be configured with a cellular, global positioning system (GPS), or other location tracking unit (not shown). The tracking unit may provide specific location of the monitoring device. This may assist in identifying specific location of the monitoring device, to the external device 324 patient monitor. The foregoing indications and responses described in conjunction with FIGS. 1 and 2 can be implemented in various combinations in other embodiments, with each indication and/or response providing its advantages even if combined with other indication(s) and/or response(s).

FIG. 4 illustrates example environment 400 including example components of a medical device 402 and example components of an external device 404 configured to perform various functions described herein. In some examples, the medical device 402 may be an AED, monitor-defibrillator, a medical imaging device, an ultrasound monitor, a standalone electrocardiogram (ECG) monitor, chest compression device, ventilator, pulse oximeter, capnograph, continuous glucose monitor (CGM), portable X-ray machine, ultrasound, non-invasive blood pressure (NIBP) monitor, a smart hospital bed or cot (e.g., a MedSurg Bed), or any other medical device configured to assess, treat, or monitor a subject.

In some examples, the medical device 402 can include one or more of input device(s) 408, sensor(s) 410, processor(s) 412, transceiver(s) 414, output device(s) 416, power source(s) 418, memory 420, malfunction detection component 422, and/or an authentication component 424. In some examples, the medical device 402 may include additional or alternative components.

In some examples, the medical device may include input device(s) 408. The input device(s) 408 may be configured to receive an input from a user and can include, for example, a button, a knob, a switch, a dial, a slider, a keypad, a cursor control, a display (e.g., including a display controller having a circuit), a voice input device (e.g., a microphone), or any combination thereof.

In some examples, the medical device 402 may include sensor(s) 410 configured to detect at least one physiological parameter of a subject. A sensor(s) 410 may include, for example, an electrode, a detection circuit, defibrillator pad, a force sensor, a blood pressure cuff, an ultrasound-based blood pressure sensor, an invasive (e.g., intra-arterial) blood pressure sensor (e.g., including a cannula inserted into the subject 124), a gas sensor (e.g., a carbon dioxide, oxygen sensor, etc.), a flowmeter, a pulse oximetry sensor, a regional oximetry sensor, a thermometer, an ultrasound transducer, and the like.

In some examples, the medical device 402 may include processor(s) 412. In some examples, the processor(s) 412 includes a central processing unit (CPU), a graphics processing unit (GPU), both CPU and GPU, or another processing unit or component known in the art. The processor(s) 412 is operably connected to the memory 420. In various implementations, the memory 420 is volatile (such as random-access memory (RAM)), nonvolatile (such as read only memory (ROM), flash memory, etc.) or some combination of the two. The memory 420 stores instructions that, when executed by the processor(s) 412, cause the processor(s) 412 to perform various operations described herein. In various examples, the memory 420 may store methods, threads, processes, applications, objects, modules, any other sort of executable instruction, or a combination thereof. In some cases, the memory 420 stores files, databases, or a combination thereof. In some examples, the memory 420 includes RAM, ROM, electrically erasable programmable read-only memory (EEPROM), flash memory, or any other memory technology. In some examples, the memory 420 includes CD-ROMs, digital versatile discs (DVDs), content-addressable memory (CAM), and/or other optical storage, magnetic cassettes, magnetic tape, magnetic disk storage and/or other magnetic storage devices, and/or any other medium (e.g., non-transitory computer-readable medium) which can be used to store the desired information and which can be accessed by the processor(s) 412 and/or the medical device 402.

In some examples, the medical device 402 may include a transceiver(s) 414. A processor may be operably connected to transceiver(s) 414. The transceiver(s) 414 may transmit and/or receive data (e.g., display data) over one or more communication networks. The transceiver(s) 414 may include a network interface card (NIC), a network adapter, a LAN adapter, or a physical, virtual, or logical address to connect to the various external devices and/or systems. In various examples, the transceiver(s) 414 includes any sort of wireless transceivers capable of engaging in wireless communication (e.g., radio frequency (RF) communication). In some examples, the transceiver(s) 414 may be configured to communicatively couple with the external device(s) via a wireless interface. For example, the communication network(s) 406 includes one or more wireless networks that include a 3rd Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 414 includes other wireless modems, such as a modem for engaging in WI-FI^{®}, WIGIG^{®}, WIMAX^{®}, BLUETOOTH^{®}, or infrared communication over communication network(s). In some examples, the medical device and/or the external device is equipped with one or more wireless communication interfaces (e.g., a radio(s), such as a Bluetooth Lowe Energy (BLE) Radio, a Wi-Fi radio, and/or a cellular radio, and so on.

In some examples, the medical device may include output device(s) 416. The output device(s) 416 may be similar to the output device(s) described with reference to FIGS. 1-3. For example, the output device(s) 416 may include a display(s) a light emitting element(s) (e.g., a LED(s)), a speaker(s), and/or a haptic actuator(s).

In some examples, the medical device may include a power source(s) 418. The power source(s) 418 may include a battery(ies), such as a primary battery that is used to carry out core functions of the medical device 402.

In some examples, the medical device 402 may include a malfunction detection component 422. The malfunction detection component 422 may be configured to detect and/or determine that a display (or a component of the display) has a malfunction. As discussed above, a malfunction may include, in some instances, a damaged touch sensor(s), backlight malfunction, discoloration of the display, freezing of the display, flickering of the display, damage of a circuit configured to power the display, a cracked display, and/or an electromagnetic interference.

In some examples, the malfunction detection component 422 may determine that a touch sensor(s) of a display 104 has a malfunction. That is, the touch sensors configured to detect a finger tap or swipe and may be defective or functioning abnormally (e.g., not responding to touch). The display of the medical device may be coated with a transparent, conductive material (e.g., indium tin oxide, fluorine doped tin oxide, niobium doped anatase TiO₂ or doped zinc oxide, etc.). The conductive material creates an electrostatic field across the display. When a finger touches the display, the finger disrupts the electrostatic field of the display. The malfunction detection component 422 is configured to measure the disruption as a change in a capacitance value and can determine the location of the touch. In some examples, the malfunction detection component 422 be configured to monitor the electrostatic field of the display and determine if the capacitance value is abnormal (e.g., by detecting inconsistencies). For example, if the electrostatic field is damaged, the malfunction detection component 422 may determine a portion of the display (or screen) has a lower-than-expected capacitance value and/or an irregular charge distribution across the display. In this example, the malfunction detection component 422 may determine that the display has a malfunction.

In some examples, a malfunction detection component 422 may be configured to monitor an electrostatic field of a display and determine if the capacitance value is abnormal (e.g., by detecting inconsistencies). For example, if the electrostatic field is damaged, the malfunction detection component 422 may determine a portion of the display (or screen) has a lower-than-expected capacitance value and/or an irregular charge distribution across the display. In this example, the malfunction detection component 422 may determine that the display has a malfunction.

In some examples, the malfunction detection component 422 may be configured to detect an electrical signal from a circuit of the display. In some examples, the malfunction detection component 422 may continuously monitor and measure a capacitance value at different points of the display. A detected lower capacitance value or a spike in a capacitance value may indicate an abnormality (e.g., a damaged trace, broken circuit, short circuit, etc.). That is, a voltage at different points of the display may drop below or exceed normal values (or range of values). The malfunction detection component 422 may determine the display has a malfunction if, for example, an overvoltage spike is detected, an undervoltage drops (e.g., due to broken traces in a display matrix), a floating voltage is detected (e.g., due to a loose connection or incomplete circuit).

In some examples, the malfunction detection component 422 may be configured to determine a circuit of the display is damaged or malfunctioning. For example, the malfunction detection component 422 may detect one or more signal(s). The signals may be associated with one or more circuits associated with a display. The signal(s) may include, for example, a power supply signal indicating voltage data and/or current for parts of the display. Voltage data may include, for example, voltage drain data indicating an operating voltage for the display driver circuit, gate high voltage and gate low voltage used to control the thin-film transistors (TFTs) of a display, common voltage data indicating reference voltage data indicating a constant voltage level used to compare the varying voltage within a pixel(s), analog voltage data, digital voltage data, backlight voltage data, and the like. In some examples, the signal(s) may include backlight control signals (e.g., pulse width modulation signals configured to control a brightness level by adjusting a duty cycle of light emitters of the display). In some examples, the signal(s) may be a pixel data signal used to transmit image information from a graphics processing unit (GPU) to a display. In some examples, the signal(s) may be a display panel control signal used to manage a liquid crystal display (LCD), including timing signals that coordinate the transfer of pixel data to the display, refresh rates, image stability, etc. In some examples, the signal(s) may include a touch input signal including analog signals that detect touch location, synchronize touch data transmission, etc. In some examples, the some instances, if a signal is taking longer to process than expected and/or if one or more signals are determined to not return at all, the malfunction detection component 422 may determine the display has a malfunction associated with a dead zone (e.g., a portion on the screen where input is not registered), a touch response is delayed, and/or an incorrect touch is registered.

In some examples, the malfunction detection component 422 may be configured to detect an electrical signal of a circuit associated with the display. In some examples, the malfunction detection component 422 may detect and/or determine a malfunction of a circuit configured to power the display. For example, the display may be associated with a display controller. In some examples, the display controller may continuously monitor and measure a capacitance value at different points of the display. A lower capacitance value or a spike in a capacitance value may indicate an abnormality such as a damaged trace, broken circuit, short circuit, etc. That is, a voltage at different points of the display may drop below or exceed normal or expected values (or range of values). The malfunction detection component 422 may determine the display has a malfunction if, for example, an overvoltage spike is detected, an undervoltage drops (e.g., due to broken traces in a display matrix), a floating voltage is detected (e.g., due to a loose connection or incomplete circuit). In some examples, the malfunction detection component 422 may be configured to determine a circuit of the display is damaged or malfunctioning when a signal is taking longer to process than expected and/or if one or more signals are determined to not return at all. In this case, the malfunction detection component 422 may determine the display has a dead zone (e.g., a portion on the screen where input is not registered), a touch response is delayed, and/or an incorrect touch is registered.

In some examples, the malfunction detection component 422 may determine the display has a backlight malfunction. For example, the display may be partially lit, with some portions of the screen being brighter than others. In some examples, the display may be flickering. The malfunction detection component 422 may determine that one or more light emitters (e.g., light emitting diodes (LEDs), cold cathode fluorescent lamps (CCFLSs), etc.) configured to light the display (or screen) are abnormal and do not illuminate properly. In some examples, the malfunction detection component 422 may determine a blacklight driver circuit configured to regulate a power to the light emitters has failed (e.g., a capacitor, resistor, transistor has failed and power is no longer received).

In some examples, the malfunction detection component 422 may determine the display has a malfunction caused by a discoloration of the display. For example, the malfunction detection component 422 may be configured to measure the red, green, and blue values of the display screen and compare the measured values to expected values. If a mismatch is detected, the malfunction detection component 422 may determine the display has a malfunction. In some examples, the malfunction detection component 422 may determine that a threshold number of the pixels of the display are defective.

In some examples, the malfunction detection component 422 may detect and/or determine an electromagnetic interference (EMI) of the display. The EMI may occur when external electromagnetic waves interfere with one or more circuits of a medical device, which may affect a function of the display (e.g., cause visual issues such as flickering, distorted images, discoloration, etc.). In some examples, the malfunction detection component 422 may determine a display is malfunctioning by measuring unexpected capacitive charge fluctuations caused by EMI.

In some examples, the medical device 402 may include an authentication component 424. The authentication component 424 may be configured to authenticate an external device prior to transmitting display data to the external device. The authentication component 424 may be configured to authenticate the external device as being authorized to mirror the display data associated with the medical device. In some examples, the authentication component 424 may be configured to store authentication information. The authentication information may verify the identity of the external device before enabling the external device to communicatively couple to the medical device. In some examples, the authentication data may be stored on a memory, such as a memory.

In some examples, the authentication component 424 may be configured to receive a request from the external device to mirror display data. In response to the request, the authentication component 424 may be configured to identify a unique identifier associated with the external device (e.g., serial number, media access control (MAC) address, etc.). The authentication component 424 compares the unique identifier to a list of authorized devices. If the external device is authenticated, the authentication component 424 may be configured to cause the external device to communicatively couple to the medical device.

In some examples, the authentication component 424 may be configured to determine that the external device is authorized to receive the display data in part by detecting that a connector is coupled with a port of the medical device and that the connector is electrically connected to the external device.

In some examples, the medical device 402 includes a reader configured to output an interrogating signal to an NFC tag of an external device 404 and receive, from the NFC tag of the external device 404 in response to the interrogating signal, a return signal. The medical device 402 may be configured to determine that the external device 404 is authorized to present the display data (e.g., mirror the display) by analyzing the return signal. In some examples, the interrogating signal includes a first electromagnetic field signal and the return signal comprises a second electromagnetic signal, and the return signal encodes an identifier of the external device 404.

In some examples, the authentication component 424 may be configured to authenticate the external device using a password-based authentication technique (e.g., the external device may be configured to send a username and password to an authentication server associated with the medical device that checks the information against a database of authorized users), certificate-based authorization technique (e.g., the external device may be presented with a digital certificate that contains a public key and a digital signature that is verified by an authentication server associated with the medical device), and the like.

The medical device 402 and the external device 404 may be configured to transmit and receive data over one or more communication networks 406. For example, the transceiver(s) 414 includes an NIC, a network adapter, a local area network (LAN) adapter, or a physical, virtual, or logical address to connect to the various external devices and/or systems. In various examples, the transceiver(s) 414 includes any sort of wireless transceivers capable of engaging in wireless communication. For example, the communication network(s) 406 includes one or more wireless networks that include a 3GPP network, such as LTE radio access network (e.g., over one or more LE bands), a NR RAN (e.g., over one or more NR bands), or a combination thereof. In some cases, the transceiver(s) 414 includes other wireless modems, such as a modem for engaging in WI-FI^{®}, WIGIG^{®}, WIMAX^{®}, BLUETOOTH^{®}, or infrared communication over the communication network(s) 406.

An external device 404 may include input device(s) 426, processor(s) 428, transceiver(s) 430, output device(s) 432, power source(s) 434, display data component 436, memory 438, and/or an application 440 including preference data 442, and/or an alert component 444. As discussed above, an external device may be a laptop, tablet, smartphone, desktop computer, smartwatch, a virtual reality headset, augmented reality glasses, and the like.

The input device(s) 426 may be the same or similar to input device(s) 408. The processor(s) 428 may be same or similar to processor(s) 412. The transceiver(s) 430 may be same or similar to transceiver(s) 414. Output device(s) 432 may be same or similar to output device(s) 416. Power source(s) 434 may be same or similar to power source(s) 418. Memory 438 may be same or similar to memory 420.

The display data component 446 may be configured to present the display data via an external display associated with the external device. In some examples, the display data component 446 may apply scaling and/or other transformation techniques to the display data. For example, if the medical device display has a lower resolution than the external display, the display data component 446 may apply interpolation techniques that generate new pixels between existing ones for smoother resolution scaling. In some examples, the display data component 446 may alter the aspect ratio of the display data (i.e., the ratio of its width to its height) prior to causing the display data to be presented via the external display.

The application 440 may be a software program including one or more instructions that are stored in a storage medium (e.g., internal memory or external memory) that is readable by a machine (e.g., external device 404). The application 440 can enable a user to specify what data (or types of data) to present via a display of the external device 404 (e.g., based on preference data 442). In some examples, the application 440 may be associated with a user profile (or account). The user profile may include preference data 442 indicating what display data is to be presented to the user via the external device 404. User preferences may include, for example, an alert preference (e.g., alert sound, alert type, visual alert, vibration or haptic feedback alert, recurring alarm, etc.), a physiological parameter preference (e.g., subscribe to receiving heart rate, blood pressure, ECG data, etc.), and the like.

In some examples, the external device 404 may include an alert component 444 that is configured to provide feedback to a rescuer (e.g., haptic feedback (e.g., vibrations, pulses, pressure changes or constriction of a band, etc.), audible feedback (e.g., notification sound or alarm), acoustic feedback, and/or visual feedback). The alert component 444 may be configured to generate an alert in response to detecting a trigger event (e.g., detecting a physiological parameter that is outside a predetermined physiological parameter range, detecting a condition or malfunction of the medical device, etc.). In response to detecting the trigger event, the alert component 444 may generate one or more of an audible alert (e.g., notification sound, alarm, etc.), haptic feedback alert, visual alert, etc.

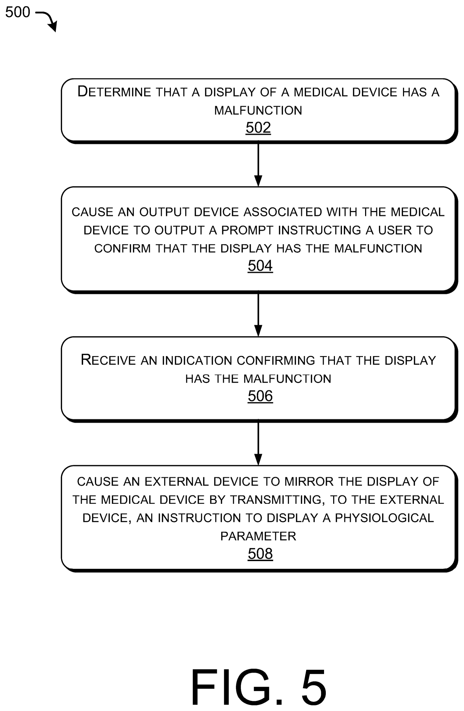

FIG. 5 illustrates an example process 500 for determining a display of a medical device has a malfunction and causing an external device to mirror the display of the medical device, according to the techniques describe herein. The process 500 may be implement by a processor or a monitoring circuit associated with a device having a display (e.g., medical device 102). For discussion purposes, the process 500 is described with reference to the previous figures.

At 502, the medical device may determine (e.g., by a processor associated with the medical device) that a display 104 is unable to visually present data (e.g., an ECG). As discussed above, in some examples, a malfunction may be a damaged touch sensor(s), backlight malfunction, discoloration of the display, freezing of the display, flickering of the display, damage of a circuit configured to power the display, a cracked display, and/or an electromagnetic interference.

At 504, in response to determining that the display has a malfunction, causing an output device associated with the medical device to output a prompt (or instruction). The prompt may be a non-visual prompt instructing a user to confirm that the display has the malfunction. In some instances, the output device may be a speaker and the prompt may be a voice prompt. That is, given that the display may be unable to visually present data, the medical device may be configured to output an instruction via a speaker that prompts the user to confirm that the display has the malfunction. For example, the voice prompt may ask a user to "confirm that the display is to be mirrored via an external display." In some examples, the instruction may prompt the user to confirm by interacting with an output device or a combination of output devices associated with the medical device. For example, the medical device may prompt the user to interact with a physical input device such as a button, a knob, a switch, a dial, a slider, or other input device on the medical device. In some examples, the medical device may generate a first prompt (e.g., a notification) configured to be displayed via the GUI of the display (if possible) and a second prompt to be output via a speaker instructing a user to confirm that the display has a malfunction.

In some instances, the malfunction may not satisfy a malfunction threshold. That is, the malfunction may not warrant causing the medical device to transmit data to an external device if the malfunction is minor and/or otherwise may not be interfering with the treatment and/or monitoring of a subject. To provide a non-limiting example, the malfunction may include a number of defective pixels or otherwise malfunctioning pixels that may result from damage to the display. For example, one or more pixels may be determined to be dead pixels (e.g., presenting as black spots), stuck pixels (e.g., pixels that are stuck on a single color), flickering pixels, and/or appearing as lines on the display. In this example, if a number of defective pixels is small (e.g., below a threshold), the medical device may not transmit display data to an external device as it may not be warranted.

In at least one example, the medical device 102 may cause a transceiver to transmit, to the external device, an instruction to display data in response to determining that a battery level associated with the medical device 102 is below a threshold level (e.g., below 20%, 10%, etc.). Displaying data on a screen can consume a significant amount of battery power. For example, higher brightness levels may require more power, larger screens with higher resolutions consumer more energy, the display may have a high refresh rate which can increase power draw, certain backlight technologies may draw more power, etc. As such, in order to preserver battery power as long as possible, the medical device 102 may be configured to transmit an instruction to the external device to display data in response to determining that a battery level is below a threshold level.

At 506, the medical device 102 may receive an indication confirming that the display has a malfunction. For example, the medical device may determine that a microphone associated with the medical device has detected an answer (e.g., "Yes, mirror the display") from a user confirming the external device is to mirror the ECG. In some examples, the medical device may determine that the user interacted with one or more physical input devices (e.g., button, knob, etc.) confirming the display has the malfunction.

At 508, the medical device may cause a transceiver to transmit an instruction to visually present a real-time (or near-real time) display data via an interface of an external device. In at least one example, the medical device may cause the transceiver to transmit multiple instructions to visually present display data via individual interfaces associated with the multiple external devices. In some examples, the instruction may cause the external de

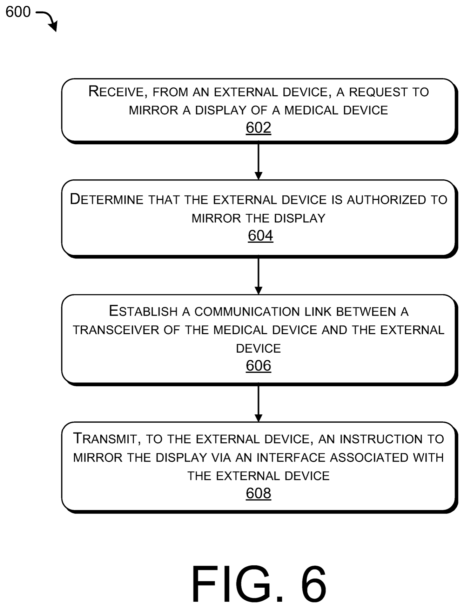

FIG. 6 illustrates an example process 600 for receiving, from an external device, a request to mirror a display of a medical device and transmitting, to the external device, an instruction to mirror the display via an interface associated with the external device. The process 600 may be implement by a processor or a monitoring circuit associated with a medical device having a display (e.g., medical device 102). For discussion purposes, the process 600 is described with reference to the previous figures.

At 602, the medical device may receive a request from an external device to mirror a display of the medical device. For instance, a user (e.g., rescuer, caregiver, etc.) may initiate pairing of the external device and the medical device.

At 604, the medical device may determine that the external device is authorized to mirror the display. In some examples, the medical device may be configured to identify and authenticate the external device. In some examples, the authentication component 424 may cause a transceiver of the medical device to output a beacon identifying the medical device (i.e., an identity of the medical device). The authentication component 424 may determine that the transceiver has received, from the external device, a response to the beacon and confirm an identify of the medical device by analyzing the response of the beacon.

At 606, the medical device may establish a communication link between a transceiver of the medical device and the external device. In some examples, the transceiver may be communicatively coupled with the external device by a wireless interface. The transceiver may be configured to transmit a stream of data packets indicating display data (e.g., measurements of physiological parameters and/or other data configured to be presented via the display of the medical device). In some examples, the interface of the external device may be configured to visually present a measurement of a physiological parameter with a time period of a sensor detecting the measurement of the physiological parameter (e.g., within 1 second, 2 seconds, etc.).

In some examples, a processor associated with the medical device may be configured to detect that a cable electrically connects the medical device to the external device. In response to detecting that the cable electrically connects the medical device and the external device, the processor transmits an instruction to the external device via the cable to visually present the real-time indication of the display data via the interface of the external device.

At 608, the medical device may transmit, to the external device, an instruction to mirror the display via an interface associated with the external device. For example, the medical device may transmit display data as communication signals (e.g., video frames, stream of data packets, etc.).

### EXAMPLE CLAUSES

1. A system including: an external device; and a monitor-defibrillator, including: a detection circuit configured to detect an electrical signal indicative of an electrocardiogram (ECG) of a subject; a transceiver; a display; and a processor configured to: determine that the display is unable to visually present the ECG; and in response to determining that the display is unable to visually present the ECG, cause the external device to mirror the ECG by causing the transceiver to transmit, to the external device, an instruction to display the ECG.
2. The system of clause 1, the monitor-defibrillator further including: a speaker; and a microphone; wherein the processor is configured to determine that the display is unable to visually present the ECG by: causing the speaker to output a voice prompt instructing a user to confirm that the display is unable to visually present the ECG; and determining that the microphone has detected an answer from the user confirming that the display is unable to visually present the ECG.
3. The system of clause 2, wherein the processor is further configured to: in response to determining that the display is unable to visually present the ECG, cause the speaker associated with the monitor-defibrillator to output an additional voice prompt instructing the user to confirm the external device is to mirror the ECG; and determine that the microphone has detected an additional answer from the user confirming that the external device is to mirror the ECG, wherein causing the transceiver to transmit, to the external device, the instruction to display the ECG is further in response to determining that the microphone has detected the answer from the user confirming that the external device is to mirror the ECG.
4. A medical device, including: a sensor configured to detect a physiological parameter of a subject; a display; a transceiver; and a processor configured to: determine that the display has a malfunction; and in response to determining that the display has the malfunction: determine that the transceiver is communicatively coupled with an external device; and cause the transceiver to transmit an instruction to visually present a real-time indication of the physiological parameter via an interface of the external device.
5. The medical device of clause 4, wherein the physiological parameter include an ECG, a an end-tidal carbon dioxide, an airway parameter, a blood oxygenation, a blood flow parameter, a blood pressure, a heart rate, or a temperature.
6. The medical device of clause 4 or 5, wherein the display includes a circuit, and wherein the processor is configured to determine that the display has the malfunction by: detecting an electrical signal from the circuit of the display; and detecting an abnormality in the electrical signal.
7. The medical device of any of clauses 4 to 6, wherein the malfunction includes: a backlight malfunction; a discoloration of the display; freezing of the display; flickering of the display; damage of a circuit configured to power the display; a cracked display; or an electromagnetic interference.
8. The medical device of any of clauses 4 to 7 further including: touch sensors integrated with the display, wherein the malfunction includes a malfunction of the touch sensors.
9. The medical device of any of clauses 4 to 8, wherein determining that the transceiver is communicatively coupled with the external device comprises: determining that the external device has been authenticated to visually present the real-time indication of the physiological parameter.
10. The medical device of any of clauses 4 to 9, further including: a connection status indicator including a light source configured to output a light signal in response to the processor causing the transceiver to transmit the instruction to visually present a real-time indication of the physiological parameter via the interface of the external device.
11. The medical device of any of clauses 4 to 10, wherein the processor is further configured to authenticate the external device by: causing the transceiver to output a beacon identifying the medical device; determining that the transceiver has received, from the external device, a response to the beacon; and confirming an identity of the external device by analyzing the response to the beacon.
12. The medical device of any of clauses 4 to 11, wherein the transceiver is communicatively coupled with the external device by a wireless interface.
13. The medical device of any of clauses 4 to 12, wherein the processor is configured to cause the transceiver to transmit an instruction by causing the transceiver to transmit a stream of data packets indicating measurements of the physiological parameter.
14. The medical device of any of clauses 4 to 13, wherein the interface of the external device visually presents a measurement of the physiological parameter within a time period of the sensor detecting the measurement of the physiological parameter.
15. The medical device of any of clauses 4 to 14, further including: a speaker configured to issue voice prompts; and an input device, wherein the processor is further configured to: generate a voice prompt instructing a user to confirm that the display has the malfunction; and determine that the input device has received, from the user, an indication confirming that the display has the malfunction.
16. The medical device of clause 15, wherein the input device includes a microphone, and wherein the indication confirming that the display has the malfunction includes a voice input from the user.
17. The medical device of clause 15, wherein the input device includes: a button, knob, switch, dial, or slider.
18. The medical device of any of clauses 4 to 17, wherein the medical device further includes: an output device; and wherein the processor is further configured to: in response to determining that the display has the malfunction, cause the output device to output a non-visual prompt instructing a user to confirm the external device is to visually present the real-time indication of the physiological parameter.
19. The medical device of any of clauses 4 to 18, wherein the processor is further configured to: detect that a cable electrically connects the medical device and the external device; and in response to detecting that the cable electrically connects the medical device and the external device, transmit, via the cable to the external device, the instruction to visually present the real-time indication of the physiological parameter via the interface of the external device.
20. A method, including: determining that a display of a medical device has a malfunction; and in response to determining that the display has the malfunction, causing an external device to mirror the display of the medical device by transmitting, to the external device, an instruction to display a physiological parameter.
21. A medical device, including: a sensor configured to detect a physiological parameter of a subject; a display; a transceiver; and a monitoring circuit configured to: determine that the display has a malfunction; and in response to determining that the display has the malfunction: determine that the transceiver is communicatively coupled with an external device; and cause the transceiver to transmit an instruction to visually present a real-time indication of the physiological parameter via an interface of the external device.
22. A system including: an external device including: a first display; and a monitor-defibrillator including: a detection circuit configured to detect an electrical signal indicative of an electrocardiogram (ECG) of a subject; a transceiver; a second display configured to visually present the ECG; and a processor configured to: receive, from the external device, a request to mirror the ECG; in response to receiving the request, determine that the external device is authorized to mirror the ECG; in response to determining that the external device is authorized to mirror the ECG, establish a communication link between the transceiver of the monitor-defibrillator and the external device; and cause the transceiver to transmit, to the external device over the communication link, an instruction to mirror the ECG via the first display, wherein the first display is configured to visually present the ECG in response to receiving the instruction to mirror the ECG.
23. The system of clause 22, the subject being a first subject, the monitor-defibrillator being a first monitor-defibrillator, the ECG being a first ECG, the system further including: a second monitor-defibrillator including: a second detection circuit configured to detect a second electrical signal indicative of a second ECG of a second subject; a second transceiver; a third display; and a second processor configured to: receive, from the external device, a request to mirror the second ECG; in response to receiving the request, determine whether the external device is authorized to mirror the second ECG; in response to determining the external device is authorized to mirror the second ECG, establish a second communication link between the second transceiver of the second monitor-defibrillator and the external device; and cause the second transceiver to transmit, to the external device over the second communication link, an instruction to mirror the second ECG via the first display, wherein the first display is configured to visually present the second ECG in response to receiving the instruction to mirror the second ECG.
24. The system of clause 23, wherein the first display is configured to: visually present an indication that the first ECG is associated with the first monitor-defibrillator or the first subject, and visually present an indication that the second ECG is associated with the second monitor-defibrillator or the second subject.
25. A medical device, including: a transceiver; a display; and a processor configured to: receive, from an external device, a request to mirror the display; in response to receiving the request, determine that the external device is authorized to mirror the display; in response to determining that the external device is authorized to mirror the display, establish a communication link between the transceiver of the medical device and the external device; and
   cause the transceiver to transmit, to the external device over the communication link, an instruction to mirror the display via an external display associated with the external device.
26. The medical device of clause 25, wherein the display is configured to visually present an indication of a physiological parameter of a subject, and wherein the instruction is to visually present the indication of the physiological parameter via the external display associated with the external device.
27. The medical device of clause 26, further including: a sensor configured to detect the physiological parameter of the subject.
28. The medical device of any of clauses 25 to 27, further including: a port, wherein determining that the external device is authorized to mirror the display comprises: detecting that a connector is coupled with the port, the connector being electrically connected to the external device.
29. The medical device of any of clauses 25 to 28, further including: a reader configured to: output an interrogating signal to a tag of the external device; and receive, from the tag of the external device in response to the interrogating signal, a return signal, wherein the processor is configured to determine that the external device is authorized to mirror the display by analyzing the return signal.
30. The medical device of clause 29, wherein the interrogating signal comprises a first electromagnetic signal and the return signal including a second electromagnetic signal, and wherein the return signal encodes an identifier of the external device.
31. The medical device of any of clauses 25 to 30, wherein establishing the communication link comprises: causing the transceiver to output a pairing request to the external device; determining that the transceiver has received a pairing response from the external device; and establishing the communication link between the transceiver and the external device in response to the pairing response.
32. The medical device of any of clauses 25 to 31, wherein the communication link is a bi-directional communication link.
33. The medical device of any of clauses 25 to 27, further including: an emitter configured to indicate that the medical device is communicatively coupled to the external device.
34. The medical device of any of clauses 25 to 33, wherein the display is a first display, the external device is a first external device, the communication link is a first communication link, and wherein the processor is configured to: receive, from a second external device, a request to mirror the first display; in response to receiving the request, determine that the second external device is authorized to mirror the first display; in response to determining that the second external device is authorized to mirror the first display, establish a second communication link between the transceiver of the medical device and the second external device; and cause the transceiver to transmit, to the second external device over the second communication link, a second instruction to mirror the first display.
35. The medical device of any of clauses 25 to 34, wherein the external device is a wearable device.
36. The medical device of any of clauses 25 to 35, wherein the external device includes a head mounted display.
37. The medical device of any of clauses 25 to 36, wherein the external device includes a first near field communication (NFC) component and the medical device includes a second NFC component, wherein the external device is configured to communicatively couple to the medical device utilizing the first NFC component and the second NFC component.
38. The medical device of any of clauses 25 to 37, wherein the external device further includes: an output device configured to output a subset of data generated by the medical device.
39. The medical device of clause 38, wherein the subset of data is determined using a type of the external device.
40. The medical device of clause 38, wherein the subset of data is output by determining a user preference associated with a user profile associated with the external device.
41. The medical device of clause 40, wherein the user preference includes: an alert preference; a physiological parameter preference; or a subscription to receive physiological data associated with one or more individuals.
42. The medical device of clause 40, wherein the user preference includes default user settings.
43. A method including: receiving, from an external device, a request to mirror a display of a medical device; in response to receiving the request, determining that the external device is authorized to mirror the display; in response to determining that the external device is authorized to mirror the display, establishing a communication link between a transceiver of the medical device and the external device; and transmitting, via the transceiver and to the external device over the communication link, an instruction to mirror the display via a user interface associated with the external device.
44. The method of clause 43, further including: presenting, via the display, an indication of a physiological parameter of a subject, and wherein the instruction is to visually present the indication of the physiological parameter via the user interface associated with the external device.
45. The method of clause 44, further including: detecting, by a sensor associated with the medical device, the physiological parameter of the subject.
46. The method of any of clauses 43 to 45, wherein determining that the external device is authorized to mirror the display includes detecting that a connector is coupled with a port disposed on the medical device, the connector being electrically connected to the external device.
47. The method of any of clauses 43 to 46, wherein establishing the communication link includes: causing the transceiver to output a pairing request to the external device; determining that the transceiver has received a pairing response from the external device; and establishing the communication link between the transceiver and the external device in response to the pairing response.
48. The method of any of clauses 43 to 47, wherein the communication link is a bi-directional communication link.
49. The method of any of clauses 43 to 48, further including: causing an emitter disposed on the medical device to emit a light, the emitter configured to indicate that the medical device is communicatively coupled to the external device.
50. The method of any of clauses 43 to 49, further including: causing an output disposed on the medical device to emit an audible tone, the audible tone configured to indicate that the medical device is communicatively coupled to the external device.
51. The method of any of clauses 43 to 50, further including: determining a battery level of the medical device; determining that the battery level is below a threshold; and in response to determining the battery level is below the threshold, transmitting, via the transceiver and to the external device over the communication link, the instruction to mirror the display via the user interface associated with the external device.

While the example clauses described above are described with respect to one particular implementation, it should be understood that, in the context of this document, the content of the example clauses can also be implemented via a method, device, system, computer-readable medium, and/or another implementation. Additionally, any one of example clauses 1-51 may be implemented alone or in combination with any other of the example clause 1-51.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A medical device, comprising: a sensor configured to detect a physiological parameter of a subject; a display; a transceiver; and a processor,
wherein the processor is configured to: determine that the display has a malfunction; and in response to determining that the display has the malfunction:
determine that the transceiver is communicatively coupled with an external device; and
cause the transceiver to transmit an instruction to visually present a real-time indication of the physiological parameter via an interface of the external device.

2. The medical device of claim 1, wherein the physiological parameter comprises an ECG, an end-tidal carbon dioxide (EtCO₂), an airway parameter, a blood oxygenation, a blood flow parameter, a blood pressure, a heart rate, or a temperature.

3. The medical device of claim 1 or 2, wherein the display comprises a circuit, and wherein the processor is configured to determine that the display has the malfunction by:
detecting an electrical signal from the circuit of the display; and
detecting an abnormality in the electrical signal.

4. The medical device of any of the preceding claims, wherein the malfunction comprises: a backlight malfunction; discoloration of the display; freezing of the display;
flickering of the display; damage of a circuit configured to power the display; a cracked display; or an electromagnetic interference.

5. The medical device of any of the preceding claims, further comprising:
touch sensors integrated with the display, wherein the malfunction comprises a malfunction of the touch sensors.

6. The medical device of any of the preceding claims, wherein determining that the transceiver is communicatively coupled with the external device comprises:
determining that the external device has been authenticated to visually present the real-time indication of the physiological parameter.

7. The medical device of any of the preceding claims, further comprising:
a connection status indicator comprising a light source configured to output a light signal in response to the processor causing the transceiver to transmit the instruction to visually present a real-time indication of the physiological parameter via the interface of the external device.

8. The medical device of any of the preceding claims, wherein the processor is further configured to authenticate the external device by:
causing the transceiver to output a beacon identifying the medical device;
determining that the transceiver has received, from the external device, a response to the beacon; and
confirming an identity of the external device by analyzing the response to the beacon.

9. The medical device of any of the preceding claims, wherein the transceiver is communicatively coupled with the external device by a wireless interface.

10. The medical device of any of the preceding claims, wherein the processor is configured to cause the transceiver to transmit an instruction by causing the transceiver to transmit a stream of data packets indicating measurements of the physiological parameter.

11. The medical device of any of the preceding claims, wherein the interface of the external device visually presents a measurement of the physiological parameter within a time period of the sensor detecting the measurement of the physiological parameter.

12. The medical device of any of the preceding claims, further comprising:
a speaker configured to issue voice prompts; and
an input device,
wherein the processor is further configured to:
generate a voice prompt instructing a user to confirm that the display has the malfunction; and
determine that the input device has received, from the user, an indication confirming that the display has the malfunction,
optionally wherein: the input device comprises a microphone, and the indication confirming that the display has the malfunction comprises a voice input from the user; or the input device comprises: a button, knob, switch, dial, or slider.

13. The medical device of any of the preceding claims, wherein the medical device further comprises:
an output device; and
wherein the processor is further configured to:
in response to determining that the display has the malfunction, cause the output device to output a non-visual prompt instructing a user to confirm the external device is to visually present the real-time indication of the physiological parameter.

14. The medical device of any of the preceding claims, wherein the processor is further configured to:
detect that a cable electrically connects the medical device and the external device; and
in response to detecting that the cable electrically connects the medical device and the external device, transmit, via the cable to the external device, the instruction to visually present the real-time indication of the physiological parameter via the interface of the external device.

15. A method, comprising:
determining that a display of a medical device has a malfunction; and
in response to determining that the display has the malfunction, causing an external device to mirror the display of the medical device by transmitting, to the external device, an instruction to display a physiological parameter.
